# EUROPÄISCHE PATENTANMELDUNG

(11) **EP 1 344 767 A2**
(43) Veröffentlichungstag der Anmeldung: **17.09.2003**
(21) Anmeldenummer: 03004809.4
(22) Anmeldetag: 04.03.2003
(51) Int. Cl.: C07C 215/40, A23K 1/16

(54) **Cholinsäuren als Futtermittelzusatz in der Tierernährung**

(30) Priorität: 11.03.2002 DE 10210642; 15.03.2002 DE 10211431
(71) Anmelder: Nutrinova Nutrition Specialties & Food Ingredients GmbH, 65929 Frankfurt am Main (DE)
(72) Erfinder: Raczek, Nico N., Dr., 65779 Kelkheim (DE); Mollenkopf, Christoph, Dr., 65929 Frankfurt (DE)
(74) Vertreter: Schweitzer, Klaus, Dr.

(57) **Zusammenfassung**

Die vorliegende Erfindung betrifft Cholinsäuren zum Einsatz als Leistungsverbesserer in der Tierernährung. Die Cholinsäuren werden synthetisiert aus Cholin und einer organischen Säure, ausgewählt aus Sorbinsäure, Benzoesäure, Propionsäure, Ameisensäure und Fumarsäure. Außerdem betrifft die Erfindung die Verwendung der Cholinsäuren allein in Futtermitteln oder in Mischung mit anderen Futterzusätzen zur Gesundheitsverbesserung.

## Beschreibung

Die Erfindung betrifft ein Produkt, welches Cholinsäuren enthält und allein in Futtermitteln oder in Abmischung mit anderen Futtermittelzusatzstoffen zur Leistungsförderung in der Tierernährung angewendet werden kann.

Leistungsförderer sind ergotrope Substanzen, die bei geringen Zulagen zum Futter die tierische Leistung, speziell das Wachstum bei jungen Nutztieren, steigern und letztlich eine günstige Futterverwertung bewirken. Leistungsfördernde Produkte sind von sehr unterschiedlicher chemischer Natur, meist mit antibiotischer Wirkung. Als Antibiotika bezeichnet man Stoffwechselprodukte bestimmter Bakterien, Pilze und zum Teil auch höherer Pflanzen, die das Wachstum vieler Mikroorganismen hemmen oder verhindern. Man spricht deshalb in der Tierernährung von einer nutritiven Anwendung der Antibiotika als Leistungsförderer, im Gegensatz zu Antibiotika, welche zu medizinischen Zwecken verabreicht werden. Im Bereich der Tierfutter werden häufig Antibiotika als Leistungsförderer verwendet. Die Anwendung von Antibiotika in diesem Bereich steht im Verdacht für Gefahren verantwortlich zu sein, welche von resistenten Bakterien ausgehen, die auch die menschliche Gesundheit langfristig gefährden können. Daher müssen gesundheitlich weniger bedenkliche Produkte für diesen Einsatzzweck gesucht werden. So werden auch in anderen Bereichen zunehmend physiologisch und epidemiologisch gesundheitlich bedenkliche oder aber für die Umwelt schädliche Substanzen wie beispielsweise Antibiotika, formaldehydabspaltende Stoffe, halogenierte Substanzen u.v.a.m. beispielsweise in Lebensmitteln, Futtermitteln, Haustierfutter, kosmetischen Mitteln, Bedarfsgegenständen, Silagen, Trester oder anderen Abfällen aus der Lebensmittelindustrie durch weniger bedenkliche Stoffe ersetzt. Der Zweck dieser Stoffe ist zum einen auf die Werterhaltung des eigentlichen Produktes gerichtet. Zum anderen aber soll auch deren hygienischer Zustand verbessert werden bzw. eine verlängerte Haltbarkeit erzielt werden.

Die Verwendung von Cholin und seinen Salzen wie Cholinchlorid ((2-Hydroxyethyl)trimethylammoniumchlorid, Formel: C₅H₁₄NOCl, MG: 139,6 g/mol, (CAS-No.: 67-48-1), gelegentlich auch als Vitamin B₄ bezeichnet, wird in der Nutztierfutterindustrie dem Futter als Vitamin zugesetzt. Für Cholin ist der Begriff "Vitamin" im klassischen Sinn aufgrund seiner Funktion und seines hohen Bedarfs im ursprünglichen Sinne jedoch kaum mehr anzuwenden. Cholin ist als lipotrope Substanz im Fettstoffwechsel beteiligt und muss daher in höherer Dosierung (g-Mengen/kg Futter) als die eigentlichen Vitamine zugeführt werden. Intermediär kann Cholin aus überschüssigem Methionin synthetisiert werden.

Cholin hat zahlreiche Funktionen im Stoffwechsel, u.a. ist es Baustein des Phosphatidylcholins und des Sphingomyelins, wichtige Teile in biologischen Membranen. Weiterhin fungiert Cholin als Methylgruppendonator und dient der Bildung von Acetylcholin (Neurotransmitterfunktion).

Cholin beeinflusst insbesondere den Fettstoffwechsel und den Membranaufbau. Eine ausreichende Cholinversorgung hat einen Spareffekt auf die Methioninzufuhr. Andererseits sinkt der Cholinbedarf bei hoher Methioninzufuhr.

Ein Cholinmangel kann auch gesundheitliche Probleme bei Tier und Mensch verursachen. Dramatische Folgen bei Cholinmangel sind besonders bei Geflügel bekannt (Fettleber). Bei Geflügel reicht die Enzymkapazität zur Methylierungsreaktion von Ethanolamin zu Cholin häufig nicht aus, um eine Versorgung durch Eigensynthese abzusichern. Daher ist eine ausreichende Cholinzufuhr über das Futter besonders bei Hühnern zu beachten. Ein weiteres Mangelsymptom ist bei wachsendem Geflügel Perosis (Abspringen der Achillessehne nach Gelenkdeformationen). Bei anderen Tieren wie beispielsweise bei Ferkeln wurden die Fehlstellungen der Hinterextremitäten bei Cholinmangel beschrieben (gespreizte Stellung).

Die Verwendung von Cholin bzw. Cholinchlorid zur Leistungssteigerung in der Nutztieraufzucht ist bereits verschiedentlich untersucht worden (Twibell, R.G. und Brown, PB., (2000). Dietary Choline Requirement of juvenile yellow perch. Journal of nutrition, 130, 95-99.; Bryant-TC; Rivera-JD; Galyean-ML; Duff-GC; Hallford-DM; Montgomery-TH, (1999). Effects of dietary level of ruminally protected choline on performance and carcass characteristics of finishing beef steers and on growth and serum metabolites in lambs. J. Animal Science 77, 2893-2903), wobei positive Effekte bei der Gewichtszunahme und der Futterverwertung festgestellt wurden (RU-A 2131678). In der Aufzucht von Jungtieren zeigten sich positive Effekte, wie beispielsweise beim Zusatz von 0,55 oder 0,88 g/kg Futter in der Ferkelaufzucht (Anonymus (1990). Sows fed choline farrow more and stronger piglets. Pigs 6, 17 und 55).

Alternative Cholinquellen aus Sojabohnen-Lecithin und daraus gewonnene entölte Lecithine enthalten bis zu 23 % Phosphatidylcholin. Beim Einsatz dieser natürlichen Cholinquelle sind jedoch keine signifikant verbesserten Leistungsparameter bei Schweinen festzustellen (Kuhn, M. et al. (1998). Utilization of crude soybean lecithin as a native choline source in feed rations of fattening pigs. Fett/Lipid 100, 78-84).

Die Anwendung von Cholinchlorid ist in der Futtermittelindustrie als Vitaminzusatz sehr verbreitet. Leider darf in Lösungen der Gehalt an Cholinchlorid ca. 75 % nicht übersteigen, da unter ca. 10° C Außentemperatur gelagerte Lösungen auskristallisieren, was große Nachteile bei der Verarbeitung, Transport und Lagerung nach sich zieht.

WO 96/08168 und US-A 6,022,566 beschreiben den Einsatz eines speziell verkapselten Cholinchlorids für Wiederkäuer. Dazu können Verbindungen, die Cholin enthalten wie Cholinbitartrat, -dihydrogencitrat, -bicarbonat, Tricholincitrat und die freie Base, verwendet werden. In WO 98/37774 werden ebenfalls mit verkapseltem Cholin oder durch Injektionen, Infusionen oder Implantate verabreichtes Cholin verbesserte Gewichtszunahmen und Futtereffektivität bei Wiederkäuern erhalten.

US-A 4,147,776 beschreibt verschiedene Cholinsalicylate zur Farbstabilisierung in pharmazeutischen Mitteln verschiedener Darreichungsformen.

EP-A 0 158 120 beschreibt ein Verfahren zur Herstellung von fließfähigen Cholinchlorid-Kieselsäure-Pulvern; EP-A 0 494 418 ein Verfahren zur Herstellung von konzentriertem Cholinchlorid auf Naturträgern zum Einsatz in Tierfuttermischungen. Auch Kombinationen aus beiden Verfahren sind bekannt (US-A-4 820 532).

Weiterhin ist bekannt, dass organische Säuren zur Konservierung von Futtermitteln eingesetzt werden können. Das Wirkprinzip wird durch den undissoziierten Säureanteil bestimmt. Die beste konservierende Wirkung entfaltet daher diese Säuren im sauren pH-Bereich.

Mit organischen Säuren wurden an verschiedenen Tierarten Fütterungsversuche durchgeführt. Diese belegen, dass verschiedene organische Säuren wie Zitronensäure, Fumarsäure oder Ameisensäure in der Lage sind, die tierischen Leistungen in positiver Weise zu beeinflussen, wenn sie in der optimalen Dosierung beispielsweise einem Ferkelfutter beigemischt werden (Kirchgeßner, M.; Roth, F.X. (1991). Ergotrope Effekte durch nutritiven Einsatz von organischen Säuren . Zbl. Hyg. 191, 265-276.; Roth, F.X.; Kirchgeßner, M. (1998). Organic acids as feed additives for young pigs: Nutritional and gastrointestinal effects. J. Anim. Feed Sci. 7, 25-33.). Weiterhin konnte auch gezeigt werden, dass beispielsweise Sorbinsäure in hohen Konzentrationen (1,8 - 2,4 % Sorbinsäure, bezogen auf das Futtermittel) eine nutritive Wirksamkeit für Aufzuchtferkel besitzt (Kirchgeßner, M.; Roth, F.X.; Paulicks, B.R., (1995). Zur nutritiven Wirkung von Sorbinsäure in der Ferkelaufzucht. J. Anim. Physiol. a. Anim. Nutr. 74, 235-242.).

Die oben beschriebenen Futtermittelzusätze sind aus den unterschiedlichsten Gründen noch nicht optimal. Es besteht nach wie vor ein Bedürfnis in der Futtermittelindustrie nach einem Futtermittelzusatz, der ausreichende Mengen an wirksamem Cholin enthält, welches problemlos zu lagern und zu verarbeiten ist. Weiterhin sollte dieser Zusatz ohne großen technischen Aufwand herzustellen sein. Außerdem soll die Haltbarkeit von Futter und Futterzusätzen über lange Zeiträume gewährleistet sein, d.h. der Futtermittelzusatz sollte auch die mikrobiologische Haltbarkeit gewährleisten. Schließlich sollte der Futtermittelzusatz einen Ersatz für antibiotische Leistungsförderer darstellen, der nicht zu einer Resistenzbildung in humanpathogenen Mikroorganismen führt.

Alle diese Aufgaben werden gelöst durch Verbindungen mit der Formel:

**Cholin**^{**+**} **X**^{**-**}

worin **Cholin**^{**+**} das Cholinkation ist und worin **X**^{**-**} Sorbat-, Benzoat-, Propionat- Formiat- oder Fumaratanion bedeutet.
**X**^{**-**} ist bevorzugt Sorbat, Propionat oder Formiat.

Diese Verbindungen werden im weiteren als Cholinsäuren bezeichnet.

Die bekannten Cholinsalze der Zitronensäure und Weinsäure zeigen nur in verkapseiter Form bei Wiederkäuern eine Wirkung und werden daher aus technologischen Gründen gegenüber dem Cholinchlorid bevorzugt. Bei den erfindungsgemäßen Cholinsäuren wird überraschender Weise nicht nur eine wachstumsfördernde Eigenschaft auch bei anderen, biologisch vollkommen andersartigen Tierarten festgestellt, sondern zusätzlich auch eine gute konservierende Wirkung erzielt. Denn schon durch Zusatz geringer Mengen eines cholinsäurehaltigen Präparates in der Ferkelaufzucht wird eine deutliche Leistungsverbesserungen hinsichtlich Zuwachsrate und Futterverwertung erreicht.

Die erfindungsgemäßen Cholinsäuren können dem Futter entweder direkt oder beispielsweise adsorbiert auf einem Träger als Futtermittelzusatzstoff beigemischt werden

Die Cholinsäuren werden zweckmäßigerweise aus einem kommerziell erhältlichen Cholinhalogenid, z.B. Cholinchlorid, und der entsprechenden Säure in Anwesenheit einer starken Base wie Natronlauge hergestellt. Alternativ lassen sie sich aus dem Cholinhalogenid und einem entsprechenden Salz der Säure wie einem Alkali- oder Erdalkalisalz unter Abtrennung des Alkali- bzw. Erdalkalihalogenids synthetisieren.

Die erfindungsgemäßen Cholinsäuren zeichnen sich durch eine leichte Handhabbarkeit aus. Sie können entweder als Reinsubstanz oder in Lösung bereitgestellt werden. Die Konzentration in wässriger Lösung beträgt dabei 40-80 %, bevorzugt 50- 80 %, besonders bevorzugt 70-80 %. Damit ist eine einfache und effektive Applizierung direkt auf das Futter bzw. die Futtervormischungen möglich.

Liegen die Cholinsäuren in Lösung vor, können dieser Lösung auch Verdickungs- und Geliermittel zur besseren Verarbeitung hinzugefügt werden. Zu Verdickungs- und Geliermitteln zählen beispielsweise: Agar-Agar, Guarkernmehl, Gummi arabicum, Johannisbrotkernmehl, Pektine, Traganth, Xanthangummi und verschiedene Cellulosen; weiterhin Sorbit, Sorbitanester und (Glycerin-Polyethylenglycol)-ricinoleat, Talgfettsäureester und Tammarindenkernmehle. Bevorzugt werden 1,2-Propandiol-Alginat, besonders bevorzugt 1,2-Propandiol, Glycerin und Alginate eingesetzt.

Überraschenderweise können konzentrierte Cholinsäurelösungen auch bei niedrigen Temperaturen noch gut verarbeitet werden. So ist beispielsweise eine 77,5 %-ige Cholinsorbatlösung bei -18 °C noch flüssig. Diese Lösung lässt sich bei 4 °C Außentemperatur problemlos auf festes Futter aufsprühen und ist gut verarbeitbar. In mikrobiologischen Untersuchungen zeigte sich weiterhin eine gute antimikrobielle Wirkung einer 75 %-igen Cholinsorbatlösung. Besonders überraschend war, dass eine synergistische Wirkung im Vergleich zum Einsatz von Kaliumsorbat und Cholinchlorid festgestellt werden konnte.

Werden die Cholinsäuren gelöst eingesetzt, können sie sowohl direkt dem Futtermittel oder einer Vormischung zugesetzt werden oder aber als Substanzlösung auf einen Träger aufgebracht werden. Als Träger können organische oder anorganische, natürliche wie synthetische saugfähige Materialien mit poröser Struktur verwendet werden. Dazu zählen beispielsweise Diatomeenerden, Sand, Tone wie Kaolin, Bentonit, Flint, Zeolithe, unlösliche Metalloxide wie Titanoxid, Aerosil, Korund, Granit, Quarz, insbesondere synthetische oder natürliche amorphe Fällungskieselsäuren, Calciumsilikat Magnesiumsilikate wie Talkum oder Natrium-Aluminiumsilikate, beispielsweise Perlit (Perlstein, E 599), unlösliche Metallsalze wie Aluminiumphosphat, Aktivkohle, Nylonpulver, Reismehl, Rübenschnitzel, Palmkernextraktionsschrot, Maismehl, Getreidekleie, Sojaextraktionsschrot, Federmehl, Fischmehl, Knochenmehl und Abfälle aus der Lebensmittelindustrie sowie Mischungen dieser Materialien.

Als Träger und zur Stabilisierung der aufgenommenen Cholinsäurelösungen werden >0 - 25 Gew.-%, bevorzugt 2,5 - 17,5 Gew.-% und besonders bevorzugt 5 - 15 Gew.-%, o.g. Trägermaterialien allein oder in Kombination, bezogen auf die entstandene Gesamtmasse (Träger + Cholinsäurelösung), eingesetzt. Die Cholinsäurelösungen werden zu diesem Zweck auf die Träger aufgesprüht, beim Mischen eingetropft oder auf dem Träger verregnet.

Die Cholinsäuren bzw. Cholinsäurelösungen oder die entsprechenden trägerfixierten Cholinsäuren/Cholinsäurelösungen (im Folgenden Cholinsäurepräparate genannt) können direkt dem Tierfuttermittel bzw. auch in Abmischung mit anderen Futtermittelzusatzstoffen oder aber über Vormischungen dem eigentlichen Futtermittel zugegeben werden.

Als Tierfuttermittel geeignet sind z. B. Grünfutter, Silagen, Trockengrünfutter, Wurzeln, Knollen, fleischige Früchte, Körner und Samen, Biertreber, Trester, Bierhefe, Destillationsschlempe, Müllereinebenerzeugnisse, Nebenerzeugnisse der Zucker-, Stärkeherstellung und Ölgewinnung und verschiedene Lebensmittelabfälle. Solchen Futtermitteln können bestimmte Futtermittelzusatzstoffe (z.B. Antioxidantien) oder Mischungen aus verschiedenen Substanzen (z.B. Mineralstoffmischungen, Vitaminmischungen) zur Verbesserung beigegeben werden. Spezielle Futtermittel werden auch für bestimmte Tierarten und deren Entwicklungsstadium angepasst. Dies ist beispielsweise in der Rinderaufzucht und der Ferkelaufzucht der Fall. Hier werden Futter für Kälber, Milchkühe, Jung- und Deckbullen und Mast- bzw. Saugferkelfutter und Ferkelaufzuchtfutter verwendet.

Die Cholinsäuren können einzeln oder im Gemisch trocken dem Futter zugemischt werden, vor einer weiteren Verarbeitung (z.B. Extrusion) zugegeben werden bzw. im Gemisch dispergiert zudosiert werden. Hierzu werden zweckmäßigerweise Cholinsäure-Konzentrationen zwischen 0,15 - 40,0 g/kg (bezogen auf das Futter), bevorzugt 0,25 - 35,0 g/kg, angewendet.

Das Cholinsäurepräparat kann als alleiniger Zusatzstoff zu Tierfuttermitteln beispielsweise für die Kälber-, Saugferkel-, Ferkel-, Geflügel-, Fisch-, Pferde- oder Lämmeraufzucht, als Aufzuchtfutter bzw. Milchaustauscherfutter, aber auch in Futter zur Mast von Geflügel, Schweinen, Wiederkäuern, Fischen und anderen Nutztieren oder bei Legehennen eingesetzt werden. Es kann auch in Abmischung mit anderen Futtermittelzusatzstoffen für diese Tiere angewendet werden. Bei der Einsatzmenge ist das Alter und der Entwicklungsstand der Tiere von entscheidender Bedeutung. Grundsätzlich sind beim aufwachsenden Tier höhere Dosen anzuwenden als bei ausgewachsenen oder in der Mast befindlichen Tieren.

Zur Sicherung einer signifikanten nutritiven Wirksamkeit ist dazu ein Zusatz an Cholinsäure in folgenden Mengen zweckmäßig:

Ferkel: 0,25 g/kg - 27,5 g/kg Futter, bevorzugt 0,5 g/kg - 17,5 g/kg Futter. Eine deutliche Leistungsverbesserungen hinsichtlich Zuwachsrate und Futterverwertung konnte auch bei der Anwendung von Cholinsäuren, insbesondere Cholinsorbat, bei der Geflügelaufzucht verzeichnet werden. Hierbei werden bei Küken 0,25 g/kg - 25 g/kg Futter, bevorzugt 0,5 g/kg - 15 g/kg Futter, dem Futter zugesetzt. Bei der Mast und Aufzucht von Truthühnern werden 0,8 - 35 g/kg Futter, bevorzugt 1,55 g/kg - 22,5 g/kg Futter, dem Futter zugesetzt. Dem Futter von Legehennen werden 0,25 g/kg - 25 g/kg Futter, bevorzugt 0,5 g/kg - 12,5 g/kg Futter, dem Futter beigegeben.

Da auch eine nutritive Wirksamkeit bei adulten Tieren festgestellt werden konnte, ist ein Zusatz an Cholinsäuren in folgenden Mengen zweckmäßig: Mastschweine 0,1 g/kg - 7,5 g/kg Futter, bevorzugt 0,2 g/kg - 5,0 g/kg Futter, und tragende Sauen 0,20 g/kg - 25 g/kg Futter, bevorzugt 0,25 g/kg - 15 g/kg Futter.

Im Folgenden wird die Erfindung anhand von Beispielen näher erläutert.

### Beispiel 1

### Herstellung von Cholinsorbat:

100 g (0,537 mol) 75 %ige Cholinchloridlösung werden vorgelegt. Dazu werden 86 g Kaliumsorbat in 86 g Wasser zugegeben. Es bildet sich ein leichter Niederschlag. Bei 50 °C und 30 mbar wird das Wasser destillativ aus dem Reaktionsgemisch entfernt. Der wachsartige Rückstand wird in 100 ml Ethanol aufgenommen. Das Reaktionsgemisch geht teilweise in Lösung. Das gebildete Kaliumchlorid fällt als Niederschlag aus und wird abgefiltert. Das Ethanol wird aus der Mutterlauge destillativ entfernt. Man erhält wieder einen wachsartigen Niederschlag, der nochmals mit 100 ml Ethanol aufgenommen wird. Es bildet sich nochmals ein Niederschlag, der ebenfalls durch Filtration abgetrennt wird. Nach Entfernen des Ethanols im Rotationsverdampfer unter Vakuum erhält man 121 g Cholinsorbat als wachsartigen Rückstand.

### Beispiel 2

Zur Herstellung einer 50 %igen Cholinsorbatlösung wird dem in Beispiel 1 genannten Ansatz 121 g Wasser zugesetzt. Man erhält eine klare Cholinsorbatlösung. Die so erhaltene Lösung kann direkt auf Futtermittel oder auf eine Vormischung aufgesprüht oder untergemischt werden.

### Beispiel 3

Das Cholinsorbat auf Trägermaterial wird hergestellt, indem man bei gleichzeitiger mechanischer Durchmischung mit Hilfe eines Taumelmischers die in Beispiel 2 gewonnene Cholinsorbatlösung langsam auf Perlit tropft. Dazu werden 10 kg Perlite in dem Mischer vorgelegt und 60 kg der Cholinsorbatlösung portionsweise in 5 kg Schritten zugegeben.

### Beispiel 4

### Herstellung von Cholinpropionat

100 g (0,537 mol) Cholinchlorid 75 % werden vorgelegt. Dazu werden 40 g Propionsäure zugegeben. Die Lösung wird mit 21,5 g Natriumhydroxid unter Kühlen versetzt. Bei 50°C und 30 mbar wird das Wasser destillativ aus dem Reaktionsgemisch entfernt. Der wachsartige Rückstand wird in 100 ml Ethanol aufgenommen. Das Reaktionsgemisch geht teilweise in Lösung. Das gebildete Natriumchlorid fällt als Niederschlag aus und wird abgefiltert. Das Ethanol wird aus der Mutterlauge destillativ entfernt. Man erhält wieder einen wachsartigen Niederschlag, der nochmals mit 100 ml Ethanol aufgenommen wird. Es bildet sich nochmals ein Niederschlag, der ebenfalls durch Filtration abgetrennt wird. Nach Entfernen des Ethanols im Rotationsverdampfer unter Vakuum erhält man 95 g Cholinpropionat.

### Beispiel 5

### Herstellung von Cholinbenzoat

100 g (0,537 mol) Cholinchlorid 75 % werden vorgelegt. Dazu werden 77,4 g Natriumbenzoat zugegeben. Bei 50°C und 30 mbar wird das Wasser destillativ aus dem Reaktionsgemisch entfernt. Der wachsartige Rückstand wird in 100 ml Ethanol aufgenommen. Das Reaktionsgemisch geht teilweise in Lösung. Das gebildete Natriumchlorid fällt als Niederschlag aus und wird abgefiltert. Das Ethanol wird aus der Mutterlauge destillativ entfernt. Man erhält wieder einen wachsartigen Niederschlag, der nochmals mit 100 ml Ethanol aufgenommen wird. Es bildet sich nochmals ein Niederschlag, der ebenfalls durch Filtration abgetrennt wird. Nach Entfernen des Ethanols im Rotationsverdampfer unter Vakuum erhält man 120 g Cholinbenzoat als Rückstand.

### Beispiel 6

### Herstellung von Cholinformiat

Die Herstellung erfolgt analog Beispiel 4. 100 g (0,537 mol) Cholinchlorid 75 % werden vorgelegt. Dazu werden 25 g Ameisensäure zugegeben. Die Lösung wird mit 21,5 g Natriumhydroxid unter Kühlen versetzt. Die weitere Aufarbeitung erfolgt wie in Beispiel 4 beschrieben. Man erhält 80 g Cholinformiat.

### Beispiel 7

### Herstellung von Natrium-Cholinfumarat

Die Herstellung erfolgt analog Beispiel 1.100 g (0,537 mol) Cholinchlorid 75 % werden vorgelegt. Dazu werden 86 g di-Natriumfumarat zugegeben. Die weitere Aufarbeitung erfolgt wie in Beispiel 1 beschrieben. Man erhält 120 g Natrium-Cholinfumarat.

### Beispiel 8

Auf ein Ferkelfutter unten genannter Zusammensetzung (Angaben in %) wird eine Cholinsorbatlösung (50 %-ig) kontinuierlich aufgesprüht, so dass durchschnittlich Gehalte von 2 g Cholinsorbat/kg erreicht werden.

| Ferkelfutter, bestehend aus (Angaben in %) | |
|---|---|
| Sojaextraktionsschrot | 22,00 |
| Gerste | 40,00 |
| Weizen | 31,00 |
| Pflanzenöl | 2,90 |
| L-Lysin - HCI | 0,40 |
| DL-Methionin | 0,10 |
| L-Threonin | 0,10 |
| Mineralfutter | 3,50 |

### Beispiel 9

Auf ein Ferkelfutter unten genannter Zusammensetzung wird eine Cholinformiatlösung (50 %-ig) kontinuierlich aufgesprüht, so dass Gehalte von 2,2 g Cholinformiat/kg Futter erreicht werden.

| Ferkelfutter bestehend aus (Angaben in %). | |
|---|---|
| Fischmehl | 4,00 |
| Sojaextraktionsschrot | 18,50 |
| Gerste | 40,00 |
| Weizen | 33,00 |
| Pflanzenöl | 1,90 |
| L-Lysin - HCI | 0,2 |
| DL-Methionin | 0,1 |
| L-Threonin | 0,1 |
| Mineralfutter | 2,2 |

### Mikrobiologische Wirksamkeit

In mikrobiologischen Untersuchungen zeigte sich weiterhin eine gute antimikrobielle Wirkung einer 75 %igen Cholinsorbatlösung. Besonders überraschend war, dass eine synergistische Wirkung im Vergleich zum Einsatz von Kaliumsorbat und Cholinchlorid festgestellt werden konnte.

In den Versuchen wurden verschiedene Schimmelpilze unter optimalen Wachstumsbedingungen angezüchtet und in einem standardisierten Agarlochtest (Hemmstofftest) die Hemmung auf die in Tabelle 1 genannten Mikroorganismen geprüft. Dazu wurden die gleichen Konzentrationen an wirksamer Substanz und daraus hergestellte wässrige 1:1-Verdünnungen jeweils in Doppelbestimmung eingesetzt. Die Angaben beziehen sich auf die totale Hemmhofgröße in mm. Übergangszonen traten nur in sehr geringem Maße auf und wurden nicht berücksichtigt.

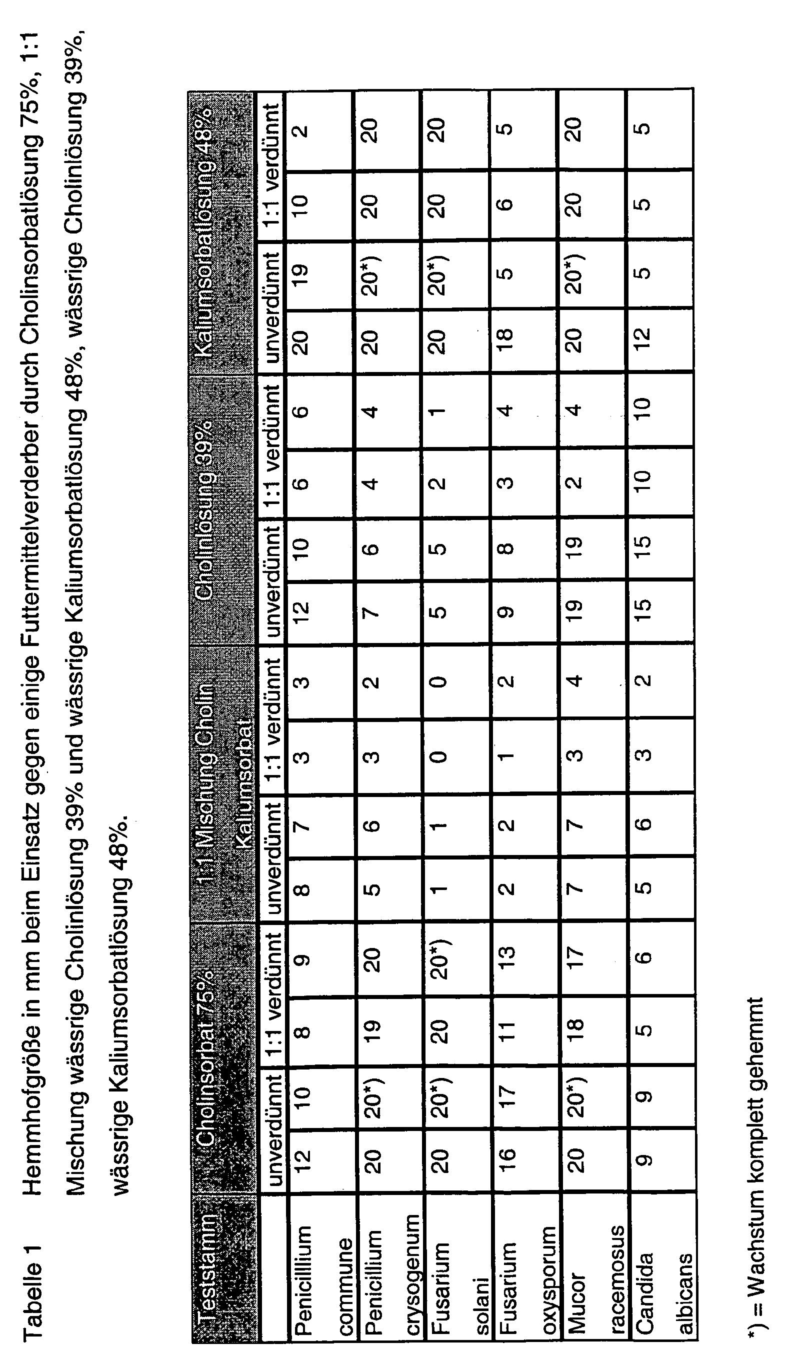

Die erhaltenen Ergebnisse belegen in eindrucksvoller Weise die hervorragende Wirkung von Cholinsorbat im Vergleich zur einfachen Mischung.

### Leistungssteigerung

Um die leistungssteigernde Wirksamkeit der erfindungsgemäßen Präparate zu untersuchen, wurden Fütterungsversuche mit je 9 Absetzferkeln (10 Tiere in Gruppe I) in Einzelhaltung durchgeführt. Das Futter der drei Versuchsgruppen war isoenergetisch zusammengesetzt und wurde den Tieren zur freien Aufnahme vorgelegt. Um Einflüsse aus Abstammung, Geschlecht oder Lebendmasse auszuschließen, wurden die Behandlungen zufällig ausgewählten Tieren zugeordnet.

Die Einmischung von Cholinsorbat erfolgte protein- und energieäquivalent, indem der Zusatz an Maisstärke und Wasser entsprechend dem Cholinsorbat-Zusatz im Verhältnis 1:1 aus den Mischungen entfernt wurde. In Tabelle 2 ist die Zusammensetzung der verwendeten Futtermischungen angegeben. Es handelt sich hierbei um praxisübliche Futtermischungen mit hohen Anteilen an Getreide, Sojaextraktionsschrot und geringen Anteilen an Fischmehl und Sojaöl. Sie entsprechen dem Standard eines Alleinfutters für Ferkel mit etwa 19 % Rohprotein, 1,14 % Lysin und 13,2 MJ umsetzbare Energie/kg Futter (Tabelle 3).

**Tabelle 2:**

| Zusammensetzung der verwendeten Futtermischungen (%) | | | |
|---|---|---|---|
| Gruppe | I | II | III |
| Weizen | 40,00 | 40,00 | 40,00 |
| Gerste | 25,55 | 25,55 | 25,55 |
| Sojaextraktionsschrot | 21,05 | 21,05 | 21,05 |
| Fischmehl | 5,00 | 5,00 | 5,00 |
| Sojaöl | 1,95 | 1,95 | 1,95 |
| Mineralfutter | 2,45 | 2,45 | 2,45 |
| Maisstärke | 2,00 | 1,90 | 1,00 |
| Wasser | 2,00 | 1,90 | 1,00 |
| Cholinsorbat (50 %ig) | - | 0,20 | 2,00 |

**Tabelle 3:**

| Nährstoffgehalte im Basisfutter (g/kg) | |
|---|---|
| Trockenmasse | 864 |
| Rohprotein | 190 |
| Rohasche | 53 |
| Rohfett | 36 |
| Rohfaser | 36 |
| N-freie Extraktstoffe | 548 |
| | |
| Umsetzbare Energie (MJ/kg) | 13,2 |
| | |
| Calcium | 8,5 |
| Phosphor | 6,4 |
| | |
| Lysin | 11,4 |
| Methionin | 3,6 |
| Methionin + Cystin | 6,8 |
| Threonin | 7,3 |
| Tryptophan | 2,4 |

Die Futtermischungen wurden den Tieren in pelletierter Form zur freien Aufnahme vorgelegt. Die Futtervorlage erfolgte täglich, das nichtverzehrte Futter wurde zweimal wöchentlich zurückgewogen. Trinkwasser stand über Selbsttränken immer zur Verfügung. Als Versuchskriterien wurden die Gewichtsentwicklung (Zuwachsrate je Tag), der Futterverzehr und die Futterverwertung (Futteraufwand je g Zuwachs) erfasst.

Bezogen auf den Gehalt an Cholinsorbat wurden Konzentrationen von 0 (kein Zusatz), 0,1 und 1,0 % dem gesamten Tierfutter zugegeben. Überraschenderweise zeigte dieser Zusatz zum Futter auch in der niedrigen Konzentration bei den Ferkeln einen deutlichen Einfluss bezogen auf das Wachstum. Zusätze steigerten die Wachstumsraten jeweils um ca. 3 %/Tag. Weiterhin ist der wirtschaftlich besonders wichtige Futterverzehr in der höchsten Dosisstufe gegenüber Gruppe I deutlich verringert. Auch die Futterverwertung war nach Cholinsorbat-Zusatz insgesamt verbessert, wobei die günstigsten Ergebnisse in der Dosisstufe 1,0 % erreicht werden konnten. Die Ergebnisse dieses Versuches sind in Tabelle 4 zusammengefasst.

**Tabelle 4:**

| Einfluss von Cholinsorbat-Zusätzen auf Lebendgewichte, Zuwachsrate, Futteraufnahme und Futterverwertung bei Ferkeln | | | | |
|---|---|---|---|---|
| Gruppe | | I | II | III |
| Cholinsorbat-Zusatz, % | | 0 | 0,1 | 1,0 |
| Anfangsgewicht, kg | | 10,01 ±1,49 | 10,02 ±1,14 | 10,05 ±1,31 |
| Endgewicht, kg | | 30,86 ±3,78 | 31,58 ±3,02 | 31,51 ±2,53 |
| | relativ | 100 | 102,3 | 102,1 |
| Zuwachsrate, g/d | | 596 ±76 | 616 ±65 | 613 ±44 |
| | relativ | 100 | 103,4 | 102,9 |
| Futterverzehr, g/d | | 925 ±116 | 957 ±84 | 899 ±66 |
| | relativ | 100 | 103,5 | 97,2 |
| Futteraufwand je g Zuwachs, g | | 1,55 ±0,06 | 1,56 ±0,09 | 1,47 ±0,10 |
| | relativ | 100 | 100,6 | 94,8 |

## Patentansprüche

1. Verbindung der Formel
**Cholin**^{**+**} **X**^{**-**}
worin **Cholin**^{**+**} das Cholinkation ist und **X**^{**-**} ein Sorbat-, Benzoat-, Propionat-, Formiat- oder Fumaratanion ist.

2. Verbindung nach Anspruch 1, **dadurch gekennzeichnet, dass X**^{**-**} ein Sorbat-, Propionat- oder Formiatanion ist.

3. Verfahren zur Herstellung einer Verbindung gemäß Anspruch 1, **dadurch gekennzeichnet, dass** man ein Cholinhalogenid mit einer Säure, ausgewählt aus Sorbinsäure, Benzoesäure, Propionsäure, Ameisensäure oder Fumarsäure oder einem Salz einer dieser Säuren umsetzt, gegebenenfalls in Anwesenheit einer Base, und anschließend gegebenenfalls das gebildete Halogenid der Base abtrennt und dann die Cholinverbindung isoliert.

4. Verwendung einer Verbindung gemäß Anspruch 1 als Futtermittelzusatzstoff oder als Zusatz zu Futtermittelvormischungen.

5. Verwendung nach Anspruch 4 in der Schweineaufzucht und -mast.

6. Verwendung nach Anspruch 4 in der Aufzucht von Zuchtsauen.

7. Verwendung nach Anspruch 4 in der Rinderaufzucht und -mast.

8. Verwendung nach Anspruch 4 in der Lämmeraufzucht und -mast.

9. Verwendung nach Anspruch 4 in der Schafzucht und -mast.

10. Verwendung nach Anspruch 4 in der Aufzucht von Küken.

11. Verwendung nach Anspruch 4 in der Aufzucht von Legehennen.

12. Verwendung nach Anspruch 4 in der Aufzucht von Truthühnern.

13. Verwendung nach Anspruch 4 in der Aufzucht und Haltung von Speisefischen.

14. Verwendung nach Anspruch 4 in der Aufzucht und Haltung von Krebstieren (Crangon spec.).

15. Verwendung nach Anspruch 4 in der Aufzucht und Haltung von Haustieren.

16. Verwendung nach Anspruch 15, bei der Aufzucht und Haltung von Hunden, Katzen, Vögeln, Aquarienfischen und Kleinnagern.

17. Präparat enthaltend einen Träger und mindestens eine darauf aufgebrachte Verbindung nach Anspruch 1.

18. Präparat nach Anspruch 17, **dadurch gekennzeichnet, dass** es >0 bis 25 Gew.-% Träger enthält.

19. Präparat nach Anspruch 17 oder 18, **dadurch gekennzeichnet, dass** der Träger ausgewählt ist aus organischen oder anorganischen, natürlichen oder synthetischen Materialien mit poröser Struktur.

20. Futtermittel, **dadurch gekennzeichnet, dass** es eine Verbindung nach Anspruch 1 enthält.

21. Futtermittel nach Anspruch 20, **dadurch gekennzeichnet, dass** es 0,15 - 40 g/kg (bezogen auf das Futtermittel) an der Verbindung enthält.

22. Futtermittel nach Anspruch 20 oder 21, **dadurch gekennzeichnet, dass** es ein Präparat nach einem der Ansprüche 17 bis 19 enthält.

23. Futtermittelzusatz, **dadurch gekennzeichnet, dass** es eine Verbindung nach Anspruch 1 enthält.

24. Futtermittelzusatz nach Anspruch 23, **dadurch gekennzeichnet, dass** es ein Präparat nach einem der Ansprüche 17 bis 19 enthält.
